# EUROPEAN PATENT APPLICATION

(11) **EP 2 011 524 A1**
(43) Date of publication of application: **07.01.2009**
(21) Application number: 07111565.3
(22) Date of filing: 02.07.2007
(51) Int. Cl.: A61L 24/10, A61L 24/04, A61L 31/04

(54) **Fibrin glue with a visualization agent**

(71) Applicant: Omrix Biopharmaceuticals Ltd., 76106 Rehovot (IL)
(72) Inventor: Nur, Israel, Moshav Timmorim (IL); Meidler, Roberto, Holon 58444 (IL); Bar, Liliana, Rehovot 76247 (IL)
(74) Representative: Meyers, Hans-Wilhelm

(57) **Abstract**

The invention relates to a fibrin glue kit, a fibrin glue fonnulation and a thrombin solution which comprise a visualization agent and to their use in methods for prevention and/or reduction of adhesions and/or methods for promotion of blood coagulation.

## Description

### Field of the invention

The invention relates to a fibrin-glue comprising a visualization agent.

### Background of the invention

Fibrin glue is a blood product obtained from either commercial sources or some regional blood transfusion centers. Valuable components for the preparation of the fibrin glue are thrombin Factor VIII, Factor XIII, fibronectin, vitronectin, von Willebrand factor (vWF), etc.

Fibrin glue is formed by an enzymatic reaction involving inter alia, fibrinogen, thrombin and Factor XIII. The thrombin converts the fibrinogen to fibrin by enzymatic action at a rate determined by the concentration of thrombin. Factor XIII, is typically present in the fibrinogen component of the glue and is an enzyme of the blood coagulation system that cross-links and stabilizes the clot's fibrin monomers. This process bypasses most of the steps of normal coagulation and mimics its last phase. Some manufacturers add anti-proteolytic agents (as described in EP Patent application 92,114,942) or specifically remove the plasminogen in order to delay the fibrinolytic action of plasmin (as described in US Patent 5,792,835 and US Patent 7,125,569). Since the fibrin clot formation is based on an enzymatic reaction, the formula which is the source of the glue, the clotting activity or physical characteristics (strength, elasticity, plasticity etc.) of the glue might be compromised by addition of chemical supplements, such as visualization agents.

The US Patent 7,009,034 discloses a composition suitable to coat a tissue of a patient comprising polymers, small molecule "crosslinkers" which remain in the cross-linked polymer and a visualization agent. The disclosed polymers are synthetic or natural. The natural polymers mentioned in the US 7,009,034 are collagen, fibrinogen, albumin, and fibrin, polysaccharides, or glycosaminoglycans. The description of US 7,009,034 is silent on Factor XIII and/or thrombin. The Examples show that addition of a visualization agent to the composition of US 7,009,034 at a high concentration of 1.2% did not cause an unacceptable change in gelation times.

According to US 7,009,034, a higher concentration of visualization agent may be used in the compositions and moreover, it is indicated that the limit of the visualization agent to be used is the solubility of the visualization agent in the final mixture. Patent application US2003/0077272 discloses proteinaceous gels having visualization agents. Disclosed are gels comprising fibrinogen and thrombin but the patent application, like in US 7,009,034, discloses cross-linking by small molecule "cross-linkers" which remain in the cross-linked polymer and is silent on Factor XIII.

### Summary of the invention

Fibrin glues are used increasingly in surgery to reduce bleeding and adhesions and to improve wound healing. The up-to-date formulations are colorless, therefore applying the preparation to the oozing site remains difficult to control. In one aspect, the present invention relates to a fibrin-glue kit or formulation comprising a visualization agent. Addition of a visualization agent improves the application targeting qualities of the fibrin glue, e.g. simplifies locating the application area, enables the user to asses the thickness of the applied material and improves the visibility of the applied material. It was found according to the present invention that addition of increased concentrations of visualization agents to the fibrin glue formulation affects thrombin clotting activity or clot formation. Also, it was found according to the invention that different visualization agents affected differently thrombin clotting activity or clot formation.

Thus, the visualization agent is added to the fibrin glue formulation or kit at a concentration which is permissive for thrombin activity and/or clot formation.

In one aspect, the invention provides a fibrin glue kit comprising at least two separate components required to form a fibrin glue; a catalyst capable of inducing cross-linking of fibrin and a visualization agent, wherein one of the at least two separated components comprises fibrinogen, and the other of the at least two separated component comprises a proteolytic enzyme which is capable of forming fibrin when it reacts with fibrinogen.

The fibrinogen and/or the catalyst capable of inducing cross-linking of fibrin and/or the proteolytic enzyme which is capable of forming fibrin can be supplied as a solution.

In an embodiment of the present invention, the catalyst is a transglutaminase such as Factor XIII.

In a further embodiment of the invention, the proteolytic enzyme is thrombin. In a further embodiment of the invention, the thrombin has a clotting activity of from about 50 to about 100% or from about 90 to about 100%.

Non limiting examples of a visualization agent in the kit or formulation of the invention are methylene blue, crystal violet, riboflavin, methyl orange, methyl red, bromothymol blue, and combinations thereof. In one embodiment of the invention, the visualization agent is methylene blue in a concentration range of from about 0.01 to about 0.2%. In another embodiment of the invention, methylene blue is in a concentration of about 0.05%.

In an embodiment of the invention, the visualization agent is incorporated in the component comprising the proteolytic enzyme.

In an embodiment of the invention, the Factor XIII is incorporated in the component comprising the fibrinogen.

In another further embodiment of the invention, the kit or formulation according to the invention is used for hemostasis and/or as anti-adhesive.

Another aspect of the invention relates to a solution comprising thrombin and a visualization agent such as methylene blue, crystal violet, riboflavin, methyl orange, methyl red, bromothymol blue, and combinations thereof. In one embodiment of the invention, the visualization agent is methylene blue. In another embodiment, the concentration of methylene blue is in the range of from about 0.01 to about 0.2%, for instance, the concentration is of about 0.05%. In still another embodiment, the clotting activity of thrombin in the solution is in the range of from about 50 to about 100% in particular in the range of from about 90 to about 100%. The solution comprising thrombin and the visualization agent can be used in fibrin-glue kit or formulation for treating hemostasis and/or treating or preventing adhesions.

One object of the present invention is to provide a method for preventing and/or reducing adhesions and/or for promoting blood coagulation by using a fibrin glue kit, a fibrin glue formulation and/or thrombin solution of the invention which comprise a visualization agent. The method is for preventing and/or reducing adhesions and/or for promoting blood coagulation and comprises administering to a subject in need a therapeutically effective amount of a fibrin glue kit, a fibrin glue formulation or composition and/or thrombin solution of the invention which comprise a visualization agent.

Another object of the invention is to provide a method of preparing a fibrin glue, for example, for surgical procedures comprising: providing a solution A-comprising fibrinogen and a catalyst capable of inducing cross-linking of fibrin; providing a solution B-comprising a proteolytic enzyme which is capable of forming fibrin when it reacts with fibrinogen and a suitable concentration of a visualization agent; applying a defined volume of the first solution A to a desired site; and applying the solution B to the site so as to cause clotting of the fibrin. Solutions A and B can be applied in any order, for example, A and B can be applied simultaneously or one after the other.

In one embodiment the proteolytic enzyme is thrombin. In another embodiment, the catalyst is a transglutaminase such as Factor XIII.

It is desirable that the clotting activity of thrombin in the fibrin glue kit, formulation of the fibrin glue and/or in the thrombin solution with the visualization agent is in the range of from about 50 to about 100%. In one embodiment, thrombin clotting activity is in the range of from about 90 to about 100%.

The visualization agent in the fibrin glue of the invention can be methylene blue, crystal violet, riboflavin, methyl orange, methyl red, bromothymol blue, and combinations thereof. In one embodiment of the invention, the visualization agent is methyene blue. Methyene blue can be used in the concentration range of from about 0.01 to about 0.2%, for example, at concentration of about 0.05%.

The fibrin glue and or thrombin solution according to the invention can be used for preventing and/or treating bleeding and/or for preventing and/or treating adhesions.

The fibrin glue obtainable according to the invention can be used for the manufacturing of a medicament for preventing or treating bleeding and/or for preventing or treating adhesions.

### Detailed description of the invention

In one aspect, the invention relates to a fibrin-glue kit or formulation comprising a visualization agent. The term fibrin glue is synonymous with the term fibrin sealant.

The fibrin glue kit of the invention comprises: at least two separate components required to form fibrin glue, fibrinogen and a proteolytic enzyme like thrombin which is capable of forming fibrin when it reacts with fibrinogen; a catalyst capable of inducing cross-linking of fibrin; and a visualization agent. The catalyst capable of inducing cross-linking of fibrin can be included in the fibrinogen component or can be in a separated component.

The term catalyst generally refers to a substance which presence increases the rate of a chemical reaction and remains substantially unchanged after completion of the respective chemical reaction in which it is involved. In one embodiment of the present invention, the catalyst is an enzyme, e.g. transglutaminase. In another embodiment, the catalyst is Factor XIII.

The fibrinogen and/or the proteolytic enzyme can be provided in the kit as a solution or in a solid form, e.g. as lyophilized powder. The solution can be in frozen state. The kit can include instructions for use.

In one embodiment of the invention, one component is comprised of fibrinogen, and a co-stabilizer such as arginine, lysine or 4-(amino methyl)-cyclo-hexane-carboxylic acid (tranexamic acid) and combinations thereof. In another embodiment, the fibrinogen component is comprised from a biologically active component (BAC) which is a solution of proteins derived from blood plasma and can comprise tranexamic acid and arginine or lysine or mixtures or arginine and lysine, or their pharmaceutically acceptable salts. In another embodiment, BAC is derived from cryoprecipitate, in particular concentrated cryoprecipitate. The solution of BAC comprises further Factor VIII, Factor XIII, fibronectin, von Willebrand factor (vWF), vitronectin), etc. Such a BAC is described in US Patent 6,121,232. In another embodiment, the BAC is free of plasminogen and plasmin as described in US Patent 7,125,569.

It is also possible that the fibrin glue formulation or kit for the fibrin glue comprises components such as thrombin, fibrinogen, Factor VIII, Factor XIII, fibronectin, vitronectin, von Willebrand factor (vWF) prepared by recombinant methods.

In one embodiment of the invention, the proteolytic enzyme which is capable to react with fibrinogen to form fibrin is thrombin, or a solution obtainable from snake venom. In one embodiment of the invention each of the components of the glue are in separated recipients such as syringes which are emptied simultaneously and a fibrin clot is formed when the components are mixed.

The invention relates to a fibrin glue formulation comprising components required to form fibrin glue, such as fibrinogen, a proteolytic enzyme like thrombin which is capable of forming fibrin when it reacts with fibrinogen; a catalyst capable of inducing cross-linking of fibrin; and a visualization agent.

The fibrinogen and/or the proteolytic enzyme can be provided in the formulation as two separated solutions prepared with a pharmaceutically acceptable carrier or in a solid form, e.g. as lyophilized powder. The solution can be in frozen state.

In an embodiment of the invention fibrinogen and the proteolytic enzyme in the formulation of the invention are in solid form and therefore need not be in separated components. The visualization agent can be formulated with one of the components before formation of the fibrin clot. In an embodiment of the invention, the visualization agent is formulated with thrombin. In another embodiment the visualization agent is formulated with the proteolytic enzyme.

Non limiting examples of visualization agents are organic dyes, food dyes and/or fluorescent dyes. If the spectrum of the visualization agent is not visible to the human eye the agent can be detected by appropriate equipment.

The invention is based on the following findings. It was found according to the present invention that addition of increased concentrations of visualization agents to the fibrin glue formulation affects thrombin clotting activity or clot formation. Also, it was found according to the invention that different visualization agents affected differently thrombin clotting activity or clot formation.

Thus, the visualization agent is added to the fibrin glue formulation or kit at a concentration which is permissive for clot formation. Clot formation is affected, for example, by the level of thrombin and/or Factor XIII activity. It is desirable that, after addition of the visualization agent to the fibrin glue formulation, the remaining thrombin clotting activity is in the range of from about 50 to about 100 %. In an embodiment, the remaining thrombin clotting activity after addition of the visualization agent is in the range of from about 90 to about 100 %.

Thrombin clotting activity can be measured directly, by the modified, European Pharmacopeia Assay (0903/1997) procedure and/or indirectly, by measuring migration length on a slanted surface (or drop test model) as described in the Examples of below, or by any other method known in the art.

The visualization agent may be chosen from various non-toxic dyes, such as methylene blue, crystal violet, riboflavin, methyl orange, methyl red, bromothymol blue, visualization agents that provide a yellow, blue, violet or orange color. In one embodiment of the invention the visualization agent is methylene blue.

The visualization agent can be in a concentration range of from about at least 0.005 to about 0.05 %, in the range of from about 0.02 to about 0.04 %, from about 0.01 to about 0.02 % or from about 0.005 to about 0.01 %.

Methylene blue can be used in a concentration in the range of from about 0.01 to about 0.2%, for example, at a concentration of about 0.05 %.

Subject matter of the present invention is also a solution comprising thrombin and a visualization agent selected from the group consisting of methylene blue, crystal violet, riboflavin, methyl orange, methyl red, bromothymol blue, and combinations thereof. It is desirable that the solution of the invention has a clotting activity of thrombin in the range of from about 50 to about 100% or of from about 90 to about 100%. In one embodiment of the invention, the visualization agent of the solution according to the invention is methylene blue and its concentration is in the range of from about 0.01 to about 0.2%, in particular about 0.05%.

Laparoscopic application of fibrin sealant by spray encompasses the worst-case conditions for targeted spray application. One of the hurdles to overcome is the effect of the laparoscopic fibrin sealant spray application on intra-abdominal pressure (IAP) and on hemodynamics. In a recent publication Druckrey-Fiskaaen et al 2007 found that fibrin sealants (Quixil) can be used safely in laparoscopic procedures if the following conditions are met: keeping the spray periods short and allowing air to escape from the abdomen. These conditions can minimize the IAP increase. According to their results, a laparoscopic spray application of fibrin sealant should start with an insufflation pressure of 10 mmHg, an application pressure of 2.5 bars, and an application distance of 5 cm with a valve on the trocar left open. This optimization of the spraying conditions opened the way to an efficient application of thin layers of the fibrin sealant in all sorts of laparoscopic applications. However, it hasn't solved the issue of targeting of the relatively thin layer of a transparent gel on a dark, internal bleeding organ. Fibrin sealant application is done very frequently under insufficient lighting when the video camera has accumulated moisture. These harsh conditions call for the spraying of a stained gel that can easily be distinguished from the surrounding tissue.

It was found according to the invention in laparoscopic application of fibrin sealant by spray that thrombin supplemented with 0.05% (500 ppm) of methylene blue was found according to the invention to be superior to that supplemented with other methylene blue concentrations. The lower methylene blue concentration (<500 ppm) was found insufficient to supply a clear targeting when thrombin and BAC were sprayed on a dark bleeding organ such as spleen or liver. Furthermore, when sprayed for short periods, fibrin glue generating thin layers where low concentrations are insufficient to allow for a confident targeting. It was found according to the invention that increasing the concentration of methylene blue might hinder the cross linking (curing) of the fibrin gel, thus the concentration of 500 ppm has the advantage that allows visibility of the sprayed gel and does not compromise the stability of the gel layer. Keeping the concentration of Methylene blue at about 500 ppm (0.05%) has been optimized to gain both.

Thus in one aspect, the invention provides laparoscopic application of a fibrin sealant comprising a visualization agent, for example by spray. In an embodiment of the invention, the visualization agent is methylene blue. In another embodiment of the invention the methylene blue is at a concentration of about 500 ppm or about 0.05%. In still another embodiment of the invention, the methylene blue is in the thrombin component of the fibrin sealant.

In another aspect, according to the invention the fibrin glue, kit, formulation or thrombin solution containing the visualization agent can be used as a hemostatic agent. The term haemostatic agent refers to the ability of the agent to stop the bleeding from an injured blood vessel and/or to contribute to keeping the blood contained within the blood vessel.

In yet another aspect, the fibrin glue, kit, formulation or thrombin solution with the visualization agent according to the invention can be used as an anti-adhesive agent. Adhesion formation is an undesired side-effect in which body tissues that are normally separated grow together. This undesirable side-effect may occur after surgical procedures, infection, trauma, or radiation. Typically, anti-adhesive agents refer to agents capable of forming a physical barrier (coating) separating between adjacent tissues at the surgical site and therefore preventing and/or reducing formation of postoperative adhesions.

The fibrin glue formulation or kit of the invention can further comprise biologically active molecules such as antibiotics, chemotherapy agents, growth factors, anti-cancer drugs. The fibrin glue or kit of the invention can be advantageously used as a drug delivery system because the visualization agent allows improved targeting qualities to the application site, for example: improves locating the targeted area, allows controlled release over an extended period and enables delivery of a required concentration which cannot be achieved in an oral delivery.

The term drug delivery system refers to delivery of bioactive molecules which are incorporated into the fibrin glue formulation or kit which allow controlled delivery of the molecules in a specific tissue in vivo.

One object of the present invention is accomplished by providing a method for preventing and/or reducing adhesions using a fibrin-glue or sealant comprising a visualization agent as mentioned above. The visualization is required in order to improve the visibility of the fibrin-glue. This characteristic enables the user to asses the thickness of the applied material. In one embodiment, the method is for treating or preventing adhesions resulting from surgical procedures, in both, presence or absence of bleeding. In another embodiment, the method is for treating or preventing adhesions of non-surgical insults such as endometriosis, infection, chemotherapy, radiation and cancer.

Another object of the invention is accomplished by providing a method for promoting coagulation of blood using a fibrin sealant with a visualization agent as specified above. The method of the invention can promote coagulation of blood of a bleeding caused as a result of surgical procedures, haemostatic disorders or in other situations where bleeding must be stopped.

Yet another object of the invention is accomplished by providing a fibrin sealant kit comprising at least two separate components required to form the fibrin glue, according to the invention, with the visualization agent and an application device. The fibrin sealant kit and applicator device can be used for the prevention and/or reduction of adhesions and/or for promoting blood coagulation or stop of bleeding.

Also subject matter of the invention is a method of preparing a fibrin glue for surgical procedures comprising providing a solution A- comprising fibrinogen and a catalyst capable of inducing cross-linking of fibrin; preparing a solution B- comprising a proteolytic enzyme which is capable of forming fibrin when it reacts with fibrinogen and a suitable concentration of a visualization agent; applying a defined volume of the solution A to a desired site; and applying solution B to the site so as to cause clotting of the fibrin. Solutions A and B can be applied in any order, for example, A and B can be applied simultaneously or one after the other. According to the invention the proteolytic enzyme is in particular thrombin, the catalyst is particularly a transglutaminase such as Factor XIII. According to the invention the clotting activity of thrombin is typically in the range of from about 50 to about 100%, particularly in the range of from about 90 to about 100%.

The visualization agent is selected from the group consisting of methylene blue, crystal violet, riboflavin, methyl orange, methyl red, bromothymol blue, and combinations thereof, in particular methylene blue in a concentration in the range of from about 0.01 to about 0.2% more particular the concentration is about 0.05%.

The disclosure of ranges in the description of the invention is easily understood by the skilled person. It means the disclosure of continuous values and figures between the limits of the ranges, including the limiting figures and values. For example, if a range is given of from 3 to 9, it is meant at least 3, 4, 5, 6, 7, 8 and or 9 with all combinations of intermediate sub ranges such as 3 and 4, 3-5, 3-6, 3-7, 3-8, or 4 and 5, 4-6, 4-7, 4-8 or 4-9 and so on.

The disclosure of applications, patents and publications, cited above or below, is hereby incorporated by reference.

The following examples are illustrative but not limiting.

### Examples

### Example 1: Effect of different dyes on thrombin clotting activity.

The present study was aimed to determine the effect of dye addition on thrombin activity. For this purpose, thrombin of a two component fibrin sealant like the one described in US Patent 6,121,232 was formulated with different dyes to final concentrations of 0.01-0.2%. The compatibility of the dyes with thrombin was tested by measuring thrombin clotting activity in the different formulations according to the following modified, European Pharmacopeia Assay (0903/1997), procedure.

Briefly, each standard solution of thrombin (4, 6, 8 and 10 IU/ml) was incubated for 2 minutes at 30°C. Then 40 µl thrombin solution of each standard solution was mixed with 160 µl fibrinogen solution (0.1 %) and clotting time was measured. A calibration curve of log clotting times vs. log thrombin concentration was plotted. Thrombin activity was determined by the clotting time obtained (calculated automatically by a clotting machine, interpolated from the calibration curve and multiplied by the dilution factor).

The following table summarizes the recovered thrombin activity in the different formulations (Table 1):

**Table 1: Recovered thrombin activity in the different formulations**

| **Dye** | **Final dye con centration (%)** | **Thrombin Activity (IU/ml)** | **Recovered activity (%)** |
|---|---|---|---|
| **Methylene Blue** | 0 | 1102 | 100 |
| | 0.01 | 1018 | 92 |
| | 0.05 | 1070 | 97 |
| | 0.1 | 991 | 90 |
| | 0.2 | 978 | 89 |
| **Crystal Violet** | 0 | 1057 | 100 |
| | 0.01 | 1125 | 106 |
| | 0.05 | 995 | 94 |
| | 0.1 | 516 | 49 |
| | 0.2 | 234 | 22 |

Methylene blue and crystal violet were tested for their effect on thrombin clotting activity at concentration 0.01-0.2%. The findings suggest that methylene blue is more compatible with thrombin than crystal violet e.g., the recovered activity of thrombin with 0.1 % methylene blue was 90 % vs. 49 % activity of a formulation with 0.1 % crystal violet. Furthermore, a formulation with 0.2% methylene blue also exhibited high recovered activity compared to crystal violet at the same concentration (89% and 22% recovered activity, respectively).

### Example 2: Effect of different dyes on clotting time.

Human thrombin was mixed with different dyes at final concentrations of 0.005-0.2%. The influence of dyes on clotting time was tested using the drop test model. Briefly, measurements of fibrin clotting time were performed on an inclined plane in a device powered by a Nitrogen pressure of 7 bars. In each experiment 5 ml of Biological Active Component (BAC), and 5ml of a thrombin solution (1000IU/ml) diluted 5 folds were pumped into a separate syringe. BAC was prepared from concentrated cryoprecipitate after being worked up as disclosed in EP-A-534 178 in which arginine and tranexamic acid were added as described in US Patent 6,121,232) These two solutions were released (about 1/8 of each) simultaneously, and a drop falls onto a slanted surface. The drop leaks down the slope until a clot is formed. The distance traveled by the drops was recorded on a millimetric paper sheet placed on the slanted surface. The distance traveled by the drop was shown to be reversibly proportional to the concentration of thrombin. The migration time of the fibrin sealant in the different formulations are listed below in Table 2.

**Table 2: Migration time of the fibrin sealant in the different formulations**

| | **Final dye concentration** | **Migration length** | **±SD (N)** | **Recoveries** |
|---|---|---|---|---|
| | **(%)** | **(cm)** | **(cm)** | |
| **Methylene Blue** | 0 | 9.4 | 0.95 (8) | 100 |
| | 0.1 | 10.7 | 2.7 (8) | 89 |
| | 0.2 | 14.3 | 0.3 (8) | 64 |
| **Crystal Violet** | 0.005 | 12.1 | 1.9 (8) | 78 |
| | 0.01 | 23.9 | 1.0 (8) | 19 |
| | 0.05 | >25 | ND (8) | 15 |
| **Riboflavin** | 0.005 | 11.5 | 1.7 (8) | 83 |
| | 0.01 | 13.3 | 1.6 (8) | 70 |
| | 0.02 | 22.9 | 2.4 (8) | 23 |
| **Methyl Or ange** | 0.005 | 8.26 | 1.6 (8) | 111 |
| | 0.01 | 7.6 | 1.2 (8) | 118 |
| **Bromothymol Blue** | 0.01 | 9.7 | 1.1 (8) | 97 |
| | 0.02 | 11.9 | 0.9 (8) | 80 |
| | | | | |

| | *Dilution | Migration length | ±SD (N) | Recoveries |
|---|---|---|---|---|
| | | (cm) | (cm) | |
| **Methyl Red** | 01:51 | 10.2 | 2.05 (8) | 93 |
| | 01:26 | 12.4 | 0.8 (8) | 76 |

| | | | | |
|---|---|---|---|---|
| *ND not determined * Methyl red was purchased from J.T. Baker (catalogue num. 5926-04) and diluted as specified above. | | | | |

In the thrombin clotting activity assay 0.01-0.05 % crystal violet did not cause interference, whereas in the drop test model crystal violet in the same range interfered with clotting activity (106 and 94 % recovered thrombin activity vs. 19 and 15 % in the drop test model). As was shown in this assay, 0.005-0.01 % riboflavin, 0.005-0.01 % methyl orange, 1:51-1:26 methyl red and 0.01- 0.02 % bromothymol blue did not interfere with clotting activity.

### Example 3: Stability of the clotting activity of fibrin-glue with the visualization agent (dye) after freeze and thaw (F&T).

Human thrombin was formulated with methylene blue, crystal violet, bromothymol blue or riboflavin at final concentrations of 0.005-0.1%. The different formulations were fast frozen to -35°C and then thawed. The effect of the freeze and thaw procedure on the stability of the glue was evaluated using the drop test model (migration time assay as in Example 2). The results are summarized in table 3.

**Table 3: Effect of the freeze and thaw procedure on the stability of the glue**

| | **Final dye concentration** | **Migration length (cm)** | **±SD (N)** | |
|---|---|---|---|---|
| | **(%)** | | **(cm)** | **Recoveries** |
| Methylene Blue | 0.1 | 10.7 | 2.7 (8) | 89 |
| | 0.1 (F&T) | 8.4 | 2.1 (8) | 110 |
| Crystal Violet | 0.005 | 12.1 | 1.9 (8) | 78 |
| | 0.005 (F&T) | 16.3 | 1.0 (9) | 52 |
| Bromothymol Blue | 0.02 | 11.9 | 0.9 (9) | 80 |
| | 0.02 (F&T) | >25 | NA(9) | 15 |
| Riboflavin | 0.005 | 11.5 | 1.7 (9) | 83 |
| | 0.005 (F&T) | 19.8 | 2 (9) | 35 |

The clotting activity of the fibrin glue formulated with methylene blue did not change significantly as a result of freezing and thawing procedure.

Thus, the experiment shows that clotting activity of the fibrin glue formulation with methylene blue is stable even though the formula was frozen and thawed.

### Example 4: Laparoscopic visibility of fibrin glue containing different concentrations of methylene blue in intraperitoneal cavity.

The objective of the experiment was to determine a suitable concentration of visualization agent in fibrin glue (as described in US Patent 6,121,232), which allows for improvement of visibility in laparoscopy during spraying on bleeding sites. Visibility of the product was determined in a pig interperitoneal laparoscopic model, on the spleen, after induction of oozing on the organ's surface. Adult female domestic crossbreed swine (n = 1) weighing 50 kg and under the age of 2 years were housed in an authorized facility according to the current ethical requirements. Anesthesia was induced with an intramuscular mixture of ketamin (15-25 mg/kg), xylazine (2 mg/kg) and di-azepam (0.5-10 mg/kg). The endotracheal tube was attached to a veterinary anesthesia machine. Anesthesia for the remainder of the preparation and surgical procedure was maintained by semi-closed circuit inhalation of isoflurane and an oxygen flow of 2 L/min. Lactated ringer's solution was administered at a rate of approximately 10 ml/kg/hr by i.p.

Physical examination was preformed prior to the surgical procedure (body weight, temperature, heart and respiratory rates). ECG, pulse, and blood pressure were monitored during the procedure.

Three laparoscopic ports were placed in the abdomen of the pig. Oozing of the spleen was induced by rubbing the organ's surface with sandpaper inserted with the laparoscope clips to the abdomen. The fibrin glue preparation was applied to the oozing site by spraying. The operator filmed the 'patient card' specifying the application number, the methylene blue concentration and time of operation. Digital video of the operation and the application site was taken before and after the application of the fibrin glue. The fibrin glue application was recorded from different angles and different zoom settings.

The dye chosen for substance coloring was methylene blue. Three different methylene blue concentrations and one non-dyed version of the fibrin glue were applied. The fibrin glue includes: 1) BAC as in Example 2 (5ml). 2) thrombin (5ml). Thrombin was supplemented with the following methylene blue concentrations: 50, 100, 500 ppm (concentrations of 0.005, 0.01, and 0.05%). The incorporation of methylene blue into the thrombin vials of the glue were performed shortly before the beginning of the operational procedure. Transportation of the vials was performed in dry ice. Vials were thawed shortly prior to use. The testing manipulation procedure was repeated for each of the testing material concentrations and the control trial.

Euthanasia was performed with an intravenous injection of KCl until heart arrest. After death has been confirmed the femoral artery was transected.

Three evaluators were appointed by the laboratory director for independent evaluation of the visibility of the fibrin glue with the different concentrations of the methylene blue. Each of the evaluators voted for the minimal methylene blue concentration which provided, to the best of his judgment, the required visibility to the fibrin glue. The evaluator took into account two parameters: 1) Color contrast between spleen surface and applied material. 2) Color contrast between blood and applied material.

All three data evaluators have independently voted for a concentration of 500 ppm of methylene blue in the thrombin vials of the glue as the minimal concentration which provides the required visibility to the fibrin glue.

These findings suggest that the fibrin glue containing 500 ppm (0.05%) of methylene blue was superior to that supplemented with other methylene blue concentrations. Lower concentrations of methylene blue (<500 ppm) were insufficient to supply a clear targeting.

It should be noted that preliminary results showed that increasing the concentration of methylene blue might hinder the cross linking of the fibrin gel. Thus, the concentration of 500 ppm is a compromise between the visibility of the sprayed gel and the stability of the gel layer. Keeping the concentration of methylene blue at 500 ppm has been optimized to gain both.

## Claims

1. A fibrin glue kit comprising: (i) at least two separate components required to form a fibrin glue (ii) a catalyst capable of inducing cross-linking of fibrin and (iii) a visualization agent, wherein one of the at least two separated components comprises fibrinogen, and the other of the at least two separated component comprises a proteolytic enzyme which is capable of forming fibrin when it reacts with fibrinogen.

2. The kit according to claim 1, wherein the fibrinogen and/or the catalyst capable of inducing cross-linking of fibrin and/or the proteolytic enzyme which is capable of forming fibrin are in solution.

3. The kit according to claim 1 and/or 2, wherein the catalyst is a transglutaminase such as Factor XIII.

4. The kit according to claim 1 to 3, wherein the proteolytic enzyme is thrombin in particular having a clotting activity of from about 50 to about 100% in particular of from about 90 to about 100%.

5. The kit according to claim 1 to 4, wherein the visualization agent is selected from the group consisting of methylene blue, crystal violet, riboflavin, methyl orange, methyl red, bromothymol blue, and combinations thereof.

6. The kit according to claim 5, wherein the concentration of methylene blue is in the range of from about 0.01 to about 0.2%, in particular is about 0.05%.

7. The kit according to any one of claims 1 to 6, wherein the visualization agent is incorporated in the component comprising the proteolytic enzyme.

8. The kit according to any one of claims 1 to 7, wherein the Factor XIII is incorporated in the component comprising the fibrinogen.

9. The kit according to any one of claims 1 to 8 for use in hemostasis and/or for use as anti-adhesive.

10. A fibrin glue formulation comprising components to form fibrin glue selected from fibrinogen, a proteolytic enzyme like thrombin which is capable of forming fibrin when it reacts with fibrinogen; and a catalyst capable of inducing cross-linking of fibrin; and a visualization agent.

11. A solution comprising thrombin and a visualization agent selected from the group consisting of methylene blue, crystal violet, riboflavin, methyl orange, methyl red, bromothymol blue, and combinations thereof.

12. The solution according to claim 11, wherein the clotting activity of thrombin is in the range of from about 50 to about 100% in particular in the range of from about 90 to about 100%.

13. The solution according to any one of claims 11 to 12, wherein the visualization agent is methylene blue.

14. The solution according to claim 13, wherein the concentration of methylene blue is in the range of from about 0.01 to about 0.2% in particular is about 0.05%.

15. A method for prevention and/or reduction of adhesions and/or for promoting blood coagulation using a kit according to any one of claims 1 to 9 or a solution according to any one o0f claims 11 to 14.

16. A method of preparing a fibrin glue comprising: preparing a solution A-comprising fibrinogen and a catalyst capable of inducing cross-linking of fibrin; preparing a solution B-comprising a proteolytic enzyme which is capable of forming fibrin when it reacts with fibrinogen and a suitable concentration of a visualization agent; applying a defined volume of solution A to a desired site; and applying the solution B to the site so as to cause clotting of the fibrin.

17. The method according to claim 16, wherein solutions A and B are applied simultaneously.

18. The method according to claim 16 or 17, wherein the proteolytic enzyme is thrombin.

19. The method according to any one of claims 16 to 18, wherein the catalyst is a transglutaminase such as Factor XIII.

20. The method according to any one of claims 16 to 19, wherein the clotting activity of thrombin is in the range of from about 50 to about 100% in particular of from about 90 to about 100%.

21. The method according to any one of claims 16 to 20, wherein the visualization agent is selected from the group consisting of methylene blue, crystal violet, riboflavin, methyl orange, methyl red, bromothymol blue, and combinations thereof.

22. The method according to claim 21, wherein the visualization agent is methyene blue at a concentration in the range of from about 0.01 to about 0.2% more particularly is about 0.05%.

23. The method according to any one of claims 16 to 22, wherein the fibrin glue is for preventing or treating bleeding and/or for preventing or treating adhesions.

24. Use of fibrin glue obtainable according to any one of the claims 16 to 22 for the manufacturing of a medicament for preventing or treating bleeding and/or for preventing or treating adhesions.
